# EUROPEAN PATENT APPLICATION

(11) **EP 4 339 157 A1**
(43) Date of publication of application: **20.03.2024**
(21) Application number: 22807437.3
(22) Date of filing: 09.05.2022
(51) Int. Cl.: C01B 33/18, C09C 3/08, A61Q 1/02, A61K 8/19, A61K 8/25, A61K 8/26, A61K 8/27, A61K 8/29

(54) **COMPOSITE SURFACE-TREATED INORGANIC POWDER**

(30) Priority: 11.05.2021 JP 2021080422
(71) Applicant: Tayca Corporation, Osaka-shi, Osaka 551-0022 (JP)
(72) Inventor: TANAKA, Toru, Osaka-shi, Osaka 551-0022 (JP); OOSAKI, Daisuke, Osaka-shi, Osaka 551-0022 (JP); SHIMIZU, Isao, Osaka-shi, Osaka 551-0022 (JP)
(74) Representative: Reitstötter Kinzebach
(86) International application number: PCT/JP2022/019687
(87) International publication number: WO 2022/239736

(57) **Abstract**

Provided is an inorganic powder which can impart feeling, which is equivalent to or greater than that of resin beads, and in particular, softness and smoothness to cosmetics. In the composite surface-treated inorganic powder, a base material powder whose main constituent includes an inorganic material or inorganic materials is surface-treated by a cationic surfactant and higher aliphatic alcohol.

## Description

### TECHNICAL FIELD

The present invention relates to inorganic powder which can be obtained by surface-treating base material powder whose main constituent includes an inorganic material or inorganic materials.

### BACKGROUND ART

Conventionally, in many cosmetics, for the purpose of enhancing feeling upon use by imparting feeling such as softness, moist feeling, and smooth feeling thereto, resin beads (a kind of microplastics) such as nylon powder and silicone beads are mixed. However, in recent years, since contamination of the ocean environment due to the microplastics has been seen as a problem, cosmetics which contain no microplastics have been demanded.

For example, described in Japanese Patent Application Laid-Open Publication No. 2008-291027 (Patent Literature 1) is an oil-in-water type emulsified composition in which powder particles like silica having a cationic surfactant adsorbed thereto are adsorbed on oil droplets dispersed in a water phase for the purpose of providing an oil-in-water type emulsified composition used in a makeup composition which has excellent emulsion stability, has no sticky feeling, and has low irritating properties.

In addition, described in International Publication No. WO2006/064821 (Patent Literature 2) is modified powder mixed in cosmetics, which is obtained by coating a surface of base powder like silicon dioxide having UV-scattering effect with octyltriethoxysilane and a cationic surfactant.

In addition, described in Japanese Patent Application Laid-Open Publication No. 2010-037328 (Patent Literature 3) is a powder cosmetic obtained by surface-treating, by a cationic active agent, a powder component or components, which contain a silicate mineral having positive ions between layers of one kind or two or more kinds selected from muscovite, phlogopite, biotite, synthetic fluorophlogopite, and silkworm mica and drying the resultant.

### CITATION LIST

### PATENT LITERATURE

Patent Literature 1: Japanese Patent Application Laid-Open Publication No. 2008-291027
Patent Literature 2: International Publication No. WO2006/064821
Patent Literature 3: Japanese Patent Application Laid-Open Publication No. 2010-037328

### SUMMARY OF THE INVENTION

### TECHNICAL PROBLEM

However, powder to be mixed in cosmetics, described in each of Patent Literatures 1 to 3, is not sufficiently satisfactory in use feeling such as softness, moist feeling, and smooth feeling, as compared with the above-mentioned resin beads, and has a problem in that softness and smoothness which are specific to the resin beads in particular cannot be obtained.

Therefore, an object of the present invention is to provide an inorganic powder which can impart feeling equivalent to or greater than that of the resin beads and in particular, softness and smoothness to cosmetics.

### SOLUTION TO PROBLEM

The present inventors have devoted themselves to earnest research. As a result, the present inventors paid attention to having softness and smoothness among the functions of the resin beads and being capable of being mixed in a water phase and an oil phase. Then, the present inventors found that by surface-treating base material powder, whose main constituent includes an inorganic material or inorganic materials, by a cationic surfactant and higher alcohol, a resin bead substitute which impart the softness and the smoothness and is capable of being mixed in a water phase and an oil phase can be obtained.

On the basis of the above-described findings, the present invention includes the following constitution. A base material powder of a composite surface-treated inorganic powder according to the present invention, whose main constituent includes an inorganic material or inorganic materials, is surface-treated by a cationic surfactant and higher aliphatic alcohol. Preferably, in the composite surface-treated inorganic powder according to the present invention, the base material powder whose main constituent includes an inorganic material or inorganic materials is surface-treated in a multi-layer structure in which layers of a hydrophilic part and a hydrophobic part of each of the cationic surfactant and the higher aliphatic alcohol, which line up in parallel with each other, are alternately stacked.

Thus, an inorganic powder which can impart feeling equivalent to or greater than that of the resin beads and in particular, softness and smoothness to cosmetics can be provided.

It is preferable that in the composite surface-treated inorganic powder according to the present invention, an emulsion viscosity ratio is 3 or more and 30 or less. In the present description, the emulsion viscosity ratio is represented by a ratio, to a viscosity of a water phase obtained by adding 10 g of the inorganic powder to 30 g of water and dispersing the resultant by a disperser for three minutes at 3000 rpm, of a viscosity of emulsion obtained by adding 10 g of hydrogenated polyisobutene to the-above-mentioned water phase and emulsifying the resultant by a homogenizing mixer for five minutes at 5000 rpm. The emulsion viscosity ratio is 3 or more and 30 or less, whereby amphipathic inorganic powder which has excellent adherability to skin and excellent feeling can be provided.

It is preferable that in the composite surface-treated inorganic powder according to the present invention, the cationic surfactant is quaternary ammonium salt which has a long-chain alkyl group having a carbon number of 8 or more and 26 or less.

It is preferable that in the composite surface-treated inorganic powder according to the present invention, the cationic surfactant is distearyl dimethyl ammonium chloride.

It is preferable that in the composite surface-treated inorganic powder according to the present invention, the higher aliphatic alcohol is alcohol which has a long-chain alkyl group having a carbon number of 18 or more and 26 or less.

It is preferable that in the composite surface-treated inorganic powder according to the present invention, the base material powder includes one or more kinds selected from the group consisting of silica, titanium oxide, zinc oxide, alumina, and iron oxide.

It is preferable that in the composite surface-treated inorganic powder according to the present invention, a surface treatment amount of the cationic surfactant is 0.1 part by mass or more and 6.0 parts by mass or less with respect to 100 parts by mass of the base material powder.

It is preferable that in the composite surface-treated inorganic powder according to the present invention, a surface treatment amount of the higher aliphatic alcohol is 0.5 part by mass or more and 8.0 parts by mass or less with respect to 100 parts by mass of the base material powder.

A cosmetic according to the present invention includes any of the above-described composite surface-treated inorganic powder.

### DESCRIPTION OF EMBODIMENT

Hereinafter, a composite surface-treated inorganic powder of the present invention will be described in detail with reference to specific examples. Note that the present invention is not limited to the below-described embodiment and a variety of modifications can be made without departing from the technical idea of the present invention.

In the composite surface-treated inorganic powder according to the present invention, a base material powder whose main constituent includes an inorganic material or inorganic materials is surface-treated by a cationic surfactant and higher aliphatic alcohol. Preferably, in the composite surface-treated inorganic powder according to the present invention, the base material powder whose main constituent includes an inorganic material or inorganic materials is surface-treated in a multi-layer structure in which layers of a hydrophilic part and a hydrophobic part of each of the cationic surfactant and the higher aliphatic alcohol, which line up in parallel with each other, are alternately stacked.

### <Base Material Powder>

The inorganic material used as the base material powder is not particularly limited and for example, one or more kinds selected from the group consisting of silica, titanium oxide, zinc oxide, alumina, and iron oxide can be used. Among these, the silica is particularly preferable.

As the base material powder, a base material powder whose average particle diameter which is measured by a laser diffraction light scattering method is in a range of 0.1 pm or more and 50 pm or less is preferable, a base material powder whose average particle diameter is in a range of 0.3 pm or more and 30 pm or less is more preferable, and a base material powder whose average particle diameter is in a range of 1 pm or more and 15 pm or less is further preferable. If the average particle diameter thereof is smaller than 0.1 pm, there may be a case where particles easily mutually agglutinate, uniform surface treatment is thereby not performed, and feeling is hardly enhanced and if the average particle diameter thereof is larger than 50 pm, there may be a case where physically rough feeling is strengthened.

A particle shape of the base material powder may be any shape as long as the shape can be ordinarily used in cosmetics, such as a spherical shape, a substantially spherical shape, a hemispherical shape, a spindle shape, a needle shape, a platy shape, a polyhedral shape, and a star shape, and a roundish shape such as the spherical shape, the substantially spherical shape, and the hemispherical shape is preferable. In addition, particles of the base material powder may be either of non-porous particles or porous particles.

### <Cationic Surfactant>

Although a kind of the cationic surfactant is not particularly limited, using a quaternary ammonium salt which has a long-chain alkyl group having a carbon number of 8 to 26 is preferable. Among these, a cationic surfactant which has two long-chain alkyl groups is more preferable.

Cited as specific examples of the cationic surfactant are distearyl dimethyl ammonium chloride, dialkyl (C12-C18) dimethyl ammonium chloride, lauryl trimethyl ammonium chloride, stearyl trimethyl ammonium chloride, cetyl trimethyl ammonium chloride, octadecyloxypropyl trimethyl ammonium chloride, behenyl trimethyl ammonium chloride, benzalkonium chloride, and the like. Among these, using the distearyl dimethyl ammonium chloride is particularly preferable.

It is preferable that a surface treatment amount of the cationic surfactant is 0.1 part by mass or more and 6.0 parts by mass or less with respect to 100 parts by mass of the base material powder, and it is more preferable that the surface treatment amount thereof is 0.1 part by mass or more and 2.0 parts by mass or less with respect thereto. Since by conducting the surface treatment in the treatment amount in this range, on a surface of the base material powder whose main constituent includes an inorganic material or inorganic materials, the multi-layer structure in which layers of the hydrophilic part and the hydrophobic part of each of the cationic surfactant and the higher aliphatic alcohol, which line up in parallel with each other, are alternately stacked is formed, a composite surface-treated inorganic powder whose feeling upon use is further favorable can be obtained. In a case where the surface treatment amount is excessively small, there may be a case where adherability to skin is not favorable and the applied cosmetic easily comes off. On the other hand, in a case where the surface treatment amount is excessively large, there may be a case where inconvenience in terms of feeling, such as stickiness upon application to skin, is caused.

### (Higher Aliphatic Alcohol)

The higher aliphatic alcohol is not limited, it is preferable that alcohol which has a long-chain alkyl group having a carbon number of 18 or more and 26 or less is used, and it is particularly preferable that behenyl alcohol or stearyl alcohol is used.

It is preferable that a surface treatment amount of the higher aliphatic alcohol is 0.5 parts by mass or more and 8.0 parts by mass or less with respect to 100 parts by mass of the base material powder, and it is more preferable that the surface treatment amount thereof is 1.0 part by mass or more and 4.0 parts by mass or less with respect thereto. Since by conducting the surface treatment in the treatment amount in this range, on a surface of the base material powder whose main constituent includes an inorganic material or inorganic materials, the multi-layer structure in which layers of the hydrophilic part and the hydrophobic part of each of the cationic surfactant and the higher aliphatic alcohol, which line up in parallel with each other, are alternately stacked is formed, feeling of the composite surface-treated inorganic powder can be made very favorable. In a case where the surface treatment amount is excessively small, there may be a case where hardness specific to the inorganic material as the base material upon application to skin is easily felt and feeling is inferior. On the other hand, in a case where the surface treatment amount is excessively large, there may be a case where for example, stickiness is felt upon the application to skin and the feeling is inferior after all.

### <Surface Treatment Method>

It is only required for a surface treatment method for obtaining the composite surface-treated inorganic powder of the present invention to be appropriately selected in consideration of physical properties of the base material powder and the surface treatment agent. For example, a method in which the base material powder is dispersed in a disperse medium, the surface treatment agent is added thereto and agitated, and thereafter, filtration and drying are conducted may be employed or a method in which the surface treatment agent is added to powder in a dried state and mixed may be employed, and the surface treatment method is not limited to these methods. Note that it is preferable that a disperse medium containing water is used when the surface treatment is conducted by the cationic surfactant.

### <Emulsion Viscosity Ratio>

It is preferable that an emulsion viscosity ratio of the composite surface-treated inorganic powder obtained by conducting the surface treatment as described above is 3 or more and 30 or less. In the present description, the emulsion viscosity ratio is represented by a ratio, to a viscosity of a water phase obtained by adding 10 g of the inorganic powder to 30 g of water and dispersing the resultant by a disperser for three minutes at 3000 rpm, of a viscosity of emulsion obtained by adding 10 g of hydrogenated polyisobutene to the-above-mentioned water phase and emulsifying the resultant by a homogenizing mixer for five minutes at 5000 rpm. The emulsion viscosity ratio is 3 or more and 30 or less, whereby the inorganic powder which has excellent adherability to skin and excellent feeling can be provided.

### <Cosmetic>

By mixing the composite surface-treated inorganic powder of the present invention to a cosmetic such as a foundation, a cosmetic which is excellent in feeling upon use and in particular, softness and smoothness can be obtained.

### [EXAMPLES]

By showing specific manufacturing examples of the composite surface-treated inorganic powder according to the present invention and test results, more detailed description will be given.

### <Example 1>

Silica powder (manufactured by AGC Si-Tech Co., Ltd.: SUNSPHERE NP-30) was added to methanol and the resultant was dispersed by conducting ultrasound irradiation for one minute and thereafter, an average particle diameter was measured by using a particle diameter distribution apparatus (manufactured by MicrotracBEL: MICROTRAC HRA). The average particle diameter was 5 pm. Also in the below-described Examples and Comparative Examples, average particle diameters of the base material powder were measured in the similar manner.

This silica powder in an amount of 100 g was dispersed in ion exchange water in an amount of 1000 g, the resultant was heated at 80°C, and distearyl dimethyl ammonium chloride having a concentration of 75% by mass (manufactured by Nikko Chemicals Co., Ltd.: NIKKOL CA-3475V, a concentration of 75% by mass) in an amount of 1.33 g and behenyl alcohol (manufactured by Nikko Chemicals Co., Ltd.: behenyl alcohol 80) in an amount of 4.0 g were inputted to 100 parts by mass of the base material powder, the resultant was agitated for 60 minutes, filtration and water washing were conducted, and thereafter, the resultant was inputted into a drying machine and dried at 85°C for 20 hours, and the resultant was pulverized, thereby obtaining a composite surface-treated inorganic powder in Example 1.

### <Example 2>

A composite surface-treated inorganic powder in Example 2 was obtained in a manner similar to the manner in which the composite surface-treated inorganic powder in Example 1 was prepared except that an inputted amount of the distearyl dimethyl ammonium chloride was 0.13 g.

### <Example 3>

A composite surface-treated inorganic powder in Example 3 was prepared in a manner similar to the manner in which the composite surface-treated inorganic powder in Example 1 was obtained except that an inputted amount of the distearyl dimethyl ammonium chloride was 0.67 g.

### <Example 4>

A composite surface-treated inorganic powder in Example 4 was prepared in a manner similar to the manner in which the composite surface-treated inorganic powder in Example 1 was obtained except that an inputted amount of the distearyl dimethyl ammonium chloride was 2.67 g.

### <Example 5>

A composite surface-treated inorganic powder in Example 5 was prepared in a manner similar to the manner in which the composite surface-treated inorganic powder in Example 1 was obtained except that the behenyl alcohol was changed to stearyl alcohol.

### <Example 6>

A composite surface-treated inorganic powder in Example 6 was prepared in a manner similar to the manner in which the composite surface-treated inorganic powder in Example 1 was obtained except that an inputted amount of the behenyl alcohol was 1.0 g.

### <Example 7>

A composite surface-treated inorganic powder in Example 7 was prepared in a manner similar to the manner in which the composite surface-treated inorganic powder in Example 1 was obtained except that an inputted amount of the behenyl alcohol was 2.0 g.

### <Example 8>

A composite surface-treated inorganic powder in Example 8 was prepared in a manner similar to the manner in which the composite surface-treated inorganic powder in Example 1 was obtained except that an inputted amount of the behenyl alcohol was 8.0 g.

### <Example 9>

A composite surface-treated inorganic powder in Example 9 was prepared in a manner similar to the manner in which the composite surface-treated inorganic powder in Example 1 was obtained except that silica powder having an average particle diameter of 1 pm (manufactured by TAYCA CORPORATION: TMS-0 1) was used.

### <Example 10>

A composite surface-treated inorganic powder in Example 10 was prepared in a manner similar to the manner in which the composite surface-treated inorganic powder in Example 1 was obtained except that silica powder having an average particle diameter of 15 pm (manufactured by TAYCA CORPORATION: TMS-15) was used.

### <Comparative Example 1>

The silica powder of the base material of the composite surface-treated inorganic powder in Example 1 (manufactured by AGC Si-Tech Co., Ltd.: SUNSPHERE NP-30) was powder in Comparative Example 1 without surface-treating the silica powder.

### <Comparative Example 2>

The silica powder, which was the base material powder in Example 1, in an amount of 100 g was dispersed in ion exchange water in an amount of 1000 g, the resultant was heated at 80°C, and thereafter, distearyl dimethyl ammonium chloride (manufactured by Nikko Chemicals Co., Ltd.: NIKKOL CA-3475V, a concentration of 75% by mass) in an amount of 1.0 g was inputted to the resultant, the resultant was agitated for 60 minutes, filtration and water washing were conducted, and thereafter, the resultant was inputted into a drying machine and dried at 85°C for 20 hours, thereby obtaining powder in Comparative Example 2.

### <Comparative Example 3>

The silica powder, which was the base material powder in Example 1, in an amount of 100 g was inputted into a table-top blender, behenyl alcohol (manufactured by Nikko Chemicals Co., Ltd.: NIKKOL behenyl alcohol 80) in an amount of 4.0 g with respect to 100 parts by mass of the base material powder was inputted, and agitation was conducted for 20 minutes, the resultant was inputted into a drying machine and dried at 120°C for three hours, and thereafter, the resultant was pulverized, thereby obtaining powder in Comparative Example 3.

### <Comparative Example 4>

Behenyl alcohol dissolved at 80°C in an amount of 0.4 g and distearyl dimethyl ammonium chloride in an amount of 0.13 g were inputted to water heated at 80°C in an amount of 30 g, the resultant was agitated by a disperser for 10 minutes, and thereafter, silica powder, which was the base material powder in Example 1, in an amount of 10 g was inputted to the resultant, and dispersion was conducted again by the disperser, thereby obtaining a water phase in Comparative Example 4.

### <Comparative Example 5>

Powder in Comparative Example 5 was obtained in a manner similar to the manner in which the composite surface-treated inorganic powder in Example 1 was obtained except that the behenyl alcohol in Example 1 was changed to octylsilane (manufactured by Momentive: SILQUEST A-137 SILANE).

### <Comparative Example 6>

Powder in Comparative Example 6 was obtained in a manner similar to the manner in which the composite surface-treated inorganic powder in Example 1 was obtained except that the behenyl alcohol in Example 1 was changed to triethylhexanoin (manufactured by The Nisshin OilliO Group, Ltd.: T.I.O).

### <Reference Example>

Powder in Reference Example was silicone resin beads (manufactured by Momentive Performance Materials: TOSPEARL (registered trademark) 145A).

### <Measurement of Emulsion Viscosity Ratios>

A viscosity of a water phase obtained by adding each powder of Examples, Comparative Examples, and Reference Example, which was made to be a constant mass by drying at 105°C for two hours, in an amount of 10 g to water in an amount of 30 g in a 150 mL-plastic container and dispersing the resultant at 3000 rpm for three minutes by a disperser (manufactured by PRIMIX Corporation, HOMOGENIZING DISPER Model 2.5, dispersion impeller (p30mm) and a viscosity of emulsion obtained by adding hydrogenated polyisobutene in an amount of 10 g to this water phase and emulsifying the resultant at 5000 rpm for five minutes by a homogenizing mixer (manufactured by PRIMIX Corporation, HOMOGENIZING MIXER MARK Model 22.5) were measured, and an emulsion viscosity ratio was obtained by a calculation formula (viscosity mPa.s after emulsifying)/(viscosity of water phase mPa·s). For the measurement of the viscosities, a B-type viscometer (manufactured by EKO Instruments Co., Ltd., Brookfield rotating viscometer LVDV-I+) was used and rotation was conducted at 12 rpm for one minute by using a spindle LV-4. Note that each of the viscosities was measured by adjusting temperatures of the emulsion and the water phase to 25°C.

### <Presence or Absence of Separation after Emulsification>

The emulsion prepared upon measuring the emulsion viscosity ratios was left to stand still under conditions of an atmospheric pressure, 25°C and one hour and thereafter, determination was visually made.

### <Evaluation of Adherability to Skin and Feeling of Each Powder>

As to each powder in each of Examples, Comparative Examples, and Reference Example, adherability to skin and feeling were evaluated by a sensory test in which five monitors participated. Specifically, the evaluation was made such that a small amount of each powder was taken and applied to the back of his or her hand by a finger, the adherability to skin and the feeling at that time were evaluated under the following criteria, and average values were calculated.

### <Criteria of Evaluation Values>

5 points: Very excellent
4 points: Excellent
3 points: Average
2 points: Inferior
1 point: Very inferior

Evaluation results (an emulsion viscosity ratio, adherability to skin, and feeling) of each powder in Examples, Comparative Examples, and Reference Example are shown in Table 1.

**[Table 1]**

| | Viscosity (mPa·s) of Water Phase | Viscosity (mPa·s) after Emulsification | Emulsion Viscosity Ratio | Presence or Absence of Separation | Adherability to Skin | Feeling |
|---|---|---|---|---|---|---|
| Example 1 | 140 | 1540 | 11.0 | Absence | 5 | 5 |
| Example 2 | 80 | 270 | 3.4 | Absence | 3 | 4 |
| Example 3 | 180 | 1370 | 7.6 | Absence | 4 | 5 |
| Example 4 | 200 | 1280 | 6.4 | Absence | 4 | 5 |
| Example 5 | 80 | 640 | 8.0 | Absence | 5 | 5 |
| Example 6 | 50 | 210 | 4.2 | Absence | 4 | 3 |
| Example 7 | 90 | 690 | 7.7 | Absence | 4 | 4 |
| Example 8 | 450 | 1500 | 3.3 | Absence | 4 | 4 |
| Example 9 | 210 | 3860 | 18.4 | Absence | 5 | 5 |
| Example 10 | 90 | 280 | 3.1 | Absence | 4 | 5 |
| Comparative Example 1 | 20 | 40 | 2.0 | Presence | 1 | 1 |
| Comparative Example 2 | 140 | 160 | 1.1 | Presence | 5 | 1 |
| Comparative Example 3 | 200 | 200 | 1.0 | Presence | 1 | 4 |
| Comparative Example 4 | 520 | 1090 | 2.1 | Presence | 1 | 1 |
| Comparative Example 5 | 60 | 60 | 1.0 | Presence | 3 | 1 |
| Comparative Example 6 | 40 | 60 | 1.5 | Presence | 3 | 1 |
| Reference Example | 320 | 120 | 0.4 | Presence | 4 | 4 |

As shown in Table 1, the composite surface-treated inorganic powder in each of Examples 1 to 10 was able to obtain adherability to skin which was equivalent to or greater than that of the powder (the silicone resin) in Reference Example which were resin beads and excellent feeling to skin upon use. Furthermore, although the powder in Reference Example separated after the emulsification, the separation after the emulsification was not observed in the composite surface-treated inorganic powder in each of Examples 1 to 10. On the other hand, as results of the powder in each of Comparative Examples 1 to 6, any of adherability to skin or feeling was inferior to that of the powder in Reference Example. In addition, the powder in each of Comparative Examples separated after the emulsification.

The described embodiment and examples are to be considered in all respects only as illustrative and not restrictive. It is intended that the scope of the invention is, therefore, indicated by the appended claims rather than the foregoing description of the embodiment and examples and that all modifications and variations coming within the meaning and equivalency range of the appended claims are embraced within their scope.

## Claims

1. A composite surface-treated inorganic powder whose base material powder is surface-treated by a cationic surfactant and higher aliphatic alcohol, a main constituent of the base material powder including an inorganic material or inorganic materials.

2. The composite surface-treated inorganic powder according to claim 1, wherein an emulsion viscosity ratio is 3 or more and 30 or less.

3. The composite surface-treated inorganic powder according to claim 1 or 2, wherein the cationic surfactant is quaternary ammonium salt which has a long-chain alkyl group having a carbon number of 8 or more and 26 or less.

4. The composite surface-treated inorganic powder according to claim 3, wherein the cationic surfactant is distearyl dimethyl ammonium chloride.

5. The composite surface-treated inorganic powder according to any one of claims 1 to 4, wherein the higher aliphatic alcohol is alcohol which has a long-chain alkyl group having a carbon number of 18 or more and 26 or less.

6. The composite surface-treated inorganic powder according to any one of claims 1 to 5, wherein the base material powder includes one or more kinds selected from the group consisting of silica, titanium oxide, zinc oxide, alumina, and iron oxide.

7. The composite surface-treated inorganic powder according to any one of claims 1 to 6, wherein a surface treatment amount of the cationic surfactant is 0.1 part by mass or more and 6.0 parts by mass or less with respect to 100 parts by mass of the base material powder.

8. The composite surface-treated inorganic powder according to any one of claims 1 to 7, wherein a surface treatment amount of the higher aliphatic alcohol is 0.5 part by mass or more and 8.0 parts by mass or less with respect to 100 parts by mass of the base material powder.

9. A cosmetic including the composite surface-treated inorganic powder according to any one of claims 1 to 8.
